# EUROPEAN PATENT APPLICATION

(11) **EP 4 509 102 A1**
(43) Date of publication of application: **19.02.2025**
(21) Application number: 23811698.2
(22) Date of filing: 16.05.2023
(51) Int. Cl.: A61F 13/02

(54) **SKIN ADHESIVE SHEET**

(30) Priority: 24.05.2022 JP 2022093999; 14.12.2022 JP 2022199529
(71) Applicant: KABUSHIKI KAISHA TOKYO MEDICAL TAPE, Tokyo 103-0022 (JP)
(72) Inventor: RIKIHISA Hiroko, Yokohama-shi, Kanagawa 221-0062 (JP)
(74) Representative: V.O.
(86) International application number: PCT/JP2023/018297
(87) International publication number: WO 2023/228824

(57) **Abstract**

With conventional skin adhesive sheets, there is a problem in that, when affixing and peeling a skin adhesive sheet to and from skin or a medical instrument, the gloves of the worker contact the adhesive surface and are torn. A skin adhesive sheet (1) according to the present disclosure comprises: a base layer (11); an adhesive (12) provided on a surface on one side of the base layer (11) and capable of adhering to a target; and a release liner (132) releasably attached to the adhesive (12). The skin adhesive sheet (1) includes: an adhesive region (171) in which the adhesive (12) is exposed as the release liner (132) is peeled and which can adhere to the target; and a non-adhesive region (172) which does not adhere to the target. The adhesive (12) is exposed by the peeling of the release liner (132), and the exposed adhesive (12) is used to complete adhesion of the base layer (11) to the target with the non-adhesive region (172) being provided thereto. After the adhesion is complete, the adhesive force of the adhesive region (171) is higher than the adhesive force of the non-adhesive region (172), and the base layer (11) can be peeled from the target by pinching the non-adhesive region (172) with fingers.

## Description

### Technical Field

The present invention relates to a skin adhesive sheet for securing an item to, for example, the human body (skin adhesive sheets described herein include those in sheet or tape form). Examples of the item include, but are not limited to, medical devices, tubes, and electrodes for electrocardiogram (ECG) monitoring that are attached to the human body for use.

### Background Art

Skin adhesive tapes have been known to be used, for example, to attach electrodes for ECG monitoring to human skin (see patent documents 1 and 2). Skin adhesive sheets with high adhesive strength have been used to secure electrodes for ECG monitoring, because the electrical activity of cardiac muscle cells may not be accurately measured if the electrodes are easily detached from the skin.

As the novel coronavirus disease (COVID-19) has spread worldwide, strict infection control measures have been implemented in the medical field. Accordingly, medical procedures that used to be performed without gloves are now performed with gloves on.

### Prior Art Document

### Patent Document

Patent Document 1: Japanese Unexamined Patent Publication No. H07-136143
Patent Document 2: Japanese Unexamined Patent Publication No. H10-272110

### Non-Patent Document

Non-Patent Document 1: Bryan Laulicht, Robert Langer, and Jeffrey M. Karp, "Quick-release medical tape", PNAS Vol. 109, No. 46, 18803-18808 (2012)

### Summary of the Invention

### Problems to be Solved by the Invention

As a result of the increase in work performed without removing gloves as described above, a new and unprecedented problem has arisen: when a healthcare worker removes a skin adhesive sheet attached to the skin while wearing gloves, the adhesive surface of the skin adhesive sheet comes into contact with the gloves, causing the skin adhesive sheet to adhere to the gloves. In addition, the gloves may tear when the healthcare worker tries to remove the skin adhesive sheet that adheres to the gloves, making it impossible to protect the healthcare worker from infection. In particular, the skin adhesive sheet is likely to adhere to the gloves when applied to the skin and also when removed from the skin after use.

It is therefore an object of the present invention to provide a skin adhesive sheet that a user can remove from the skin or a medical device without touching its adhesive surface while wearing gloves. In particular, it is an object to provide a skin adhesive sheet that a user can remove from the skin or a medical device without touching its adhesive surface while wearing gloves when it is removed from the skin after application and use.

### Means for Solving the Problems

To achieve the object mentioned above, according to the first aspect of the invention, a skin adhesive sheet includes: a base layer; an adhesive that is provided on a surface on one side of the base layer and can adhere to an object; and a release liner that is removably attached to the adhesive. The skin adhesive sheet has an adhesive region that can adhere to the object when the adhesive is exposed by removal of the release liner and a non-adhesive region that does not adhere to the object. The release liner is removed to expose the adhesive so that the base layer can be adhered to the object with the exposed adhesive while having the non-adhesive region. The adhesive region has a higher adhesive strength than the non-adhesive region after adhesion is complete. The non-adhesive region can be pinched with fingers to remove the base layer from the object.

According to the second aspect of the invention, in the skin adhesive sheet of the first aspect, the base layer is adhered to the object while having the non-adhesive region to thereby secure a specific item to the object, and the non-adhesive region can be pinched with fingers to remove the base layer from the object, allowing the specific item to be removed from the object.

According to the third aspect of the invention, in the skin adhesive sheet of the first or second aspect, the non-adhesive region is located at a position along the outer edges of the base layer.

According to the fourth aspect of the invention, in the skin adhesive sheet of the first or second aspect, the non-adhesive region is formed by a covering that is attached to the base layer through the adhesive, and the covering is not removed from the base layer when the release liner is removed to apply the skin adhesive sheet to the object.

According to the fifth aspect of the invention, in the skin adhesive sheet of the first or second aspect, the skin adhesive sheet is rectangular, nearly rectangular, strip-shaped, or nearly strip-shaped, and the non-adhesive region is not present in a central region that includes a center line connecting a first midpoint at the center of a first long side of the skin adhesive sheet and a second midpoint at the center of a second long side of the skin adhesive sheet.

According to the sixth aspect of the invention, in the skin adhesive sheet of the first or second aspect, the skin adhesive sheet is elliptical or nearly elliptical, and the non-adhesive region is not present in a central region that includes the minor axis of the skin adhesive sheet.

According to the seventh aspect of the invention, in the skin adhesive sheet of the fourth aspect, the base layer has a first side, a second side, and a base layer corner defined by the first side and the second side. The covering has a first side to be along the first side of the base layer, a second side to be along the second side of the base layer, a covering first corner defined by the first side and the second side and to be aligned with the base layer corner, and a covering second corner located opposite the covering first corner on the first side. The adhesive region includes a side protection zone as part thereof in between the covering second corner and the first side of the base layer, and the side protection zone reduces irritation to the object caused by the covering second corner when the skin adhesive sheet is applied to the object.

According to the eighth aspect of the invention, in the skin adhesive sheet of the seventh aspect, the covering second corner is rounded.

According to the ninth aspect of the invention, in the skin adhesive sheet of the eighth aspect, the covering second corner has a radius of curvature of 0.1 mm or more and 5.0 mm or less.

According to the tenth aspect of the invention, in the skin adhesive sheet of the seventh aspect, the covering is triangular in shape. The covering further has a covering third corner located opposite the covering first corner on the second side, and the covering third corner has a radius of curvature of 0.1 mm or more and 5.0 mm or less. The adhesive region further includes a second side protection zone as part thereof in between the covering third corner and the second side of the base layer, and the second side protection zone reduces irritation to the object caused by the covering third corner when the skin adhesive sheet is applied to the object.

According to the eleventh aspect of the invention, in the skin adhesive sheet of the seventh aspect, the base layer further has a third side opposite the first side, and the covering is rectangular in shape. The covering further has a third side to be along the third side of the base layer, and a covering third corner located at a position continuous with the third side and diagonal to the covering first corner. The covering third corner has a radius of curvature of 0.1 mm or more and 5.0 mm or less. The adhesive region further includes a second side protection zone as part thereof in between the covering third corner and the third side of the base layer, and the second side protection zone reduces irritation to the object caused by the covering third corner when the skin adhesive sheet is applied to the object.

According to the twelfth aspect of the invention, in the skin adhesive sheet of the seventh aspect, a side of the covering facing a side of the release liner has a recessed portion or a raised portion.

### Effects of the Invention

According to certain aspects, the present invention may provide a skin adhesive sheet that a user can remove from the skin or a medical device without touching its adhesive surface while wearing gloves. In particular, the invention may provide a skin adhesive sheet that a user can remove from the skin or a medical device without touching its adhesive surface while wearing gloves when it is removed from the skin after application and use.

### Brief Description of the Drawings

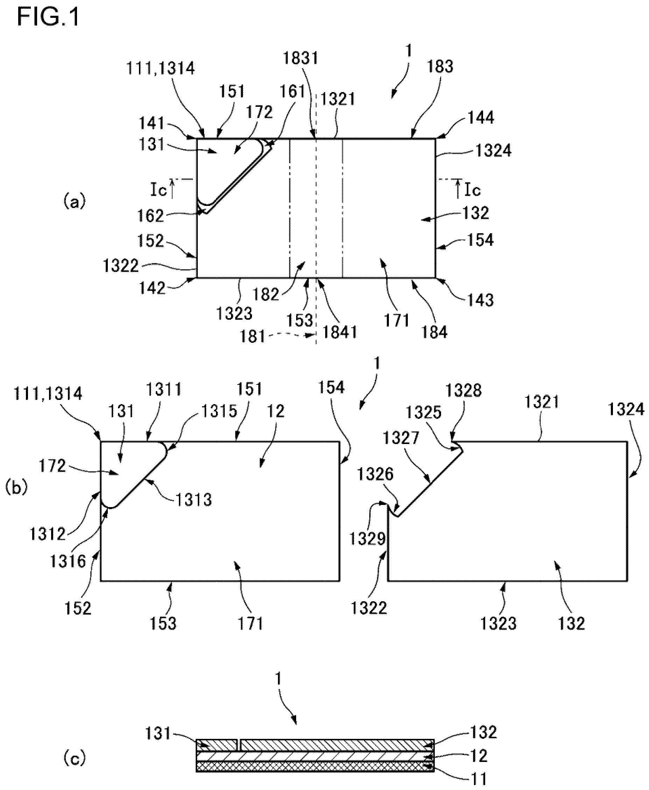

[FIG. 1] FIG. 1 includes diagrams illustrating a configuration of a skin adhesive sheet according to a first embodiment of the invention: FIG. 1(a) is a top view of the entire skin adhesive sheet as viewed from the release liner side, the left side of FIG. 1(b) is a top view of the base layer and covering, the right side of FIG. 1(b) is a top view of the release liner, and FIG. 1(c) is a cross-sectional view taken along line Ic-Ic in FIG. 1(a).

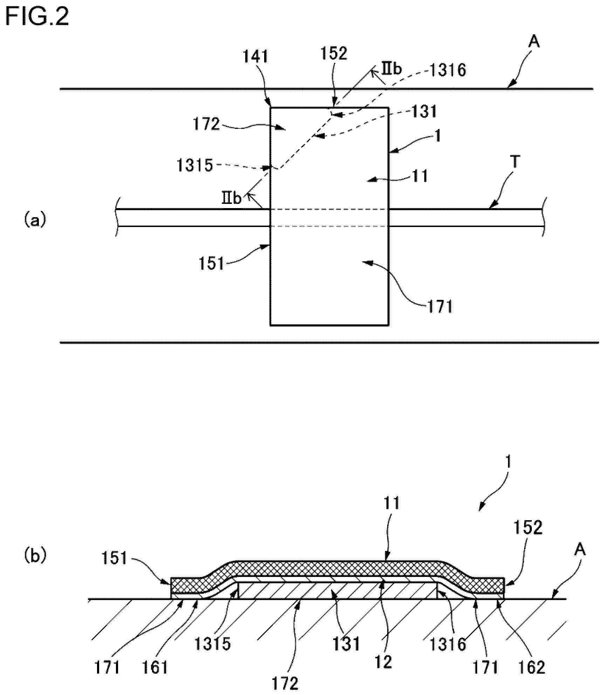

[FIG. 2] FIG. 2 includes diagrams illustrating a state in which the skin adhesive sheet is applied to an object: FIG. 2(a) is a top view, and FIG. 2(b) is a cross-sectional view taken along line IIb-IIb in FIG. 2(a).

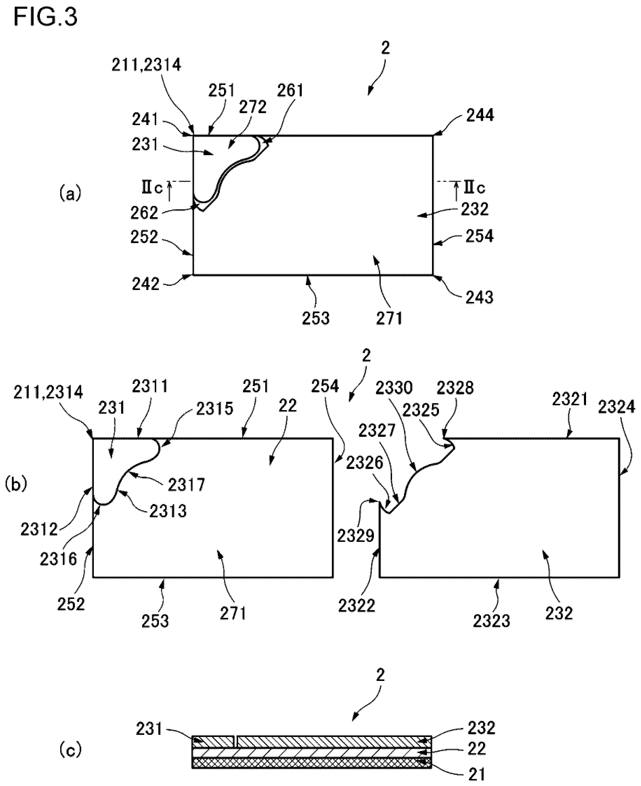

[FIG. 3] FIG. 3 includes diagrams illustrating a configuration of a skin adhesive sheet according to a second embodiment of the invention: FIG. 3(a) is a top view of the entire skin adhesive sheet as viewed from the release liner side, the left side of FIG. 3(b) is a top view of the base layer and covering, the right side of FIG. 3(b) is a top view of the release liner, and FIG. 3(c) is a cross-sectional view taken along line IIc-IIc in FIG. 3(a).

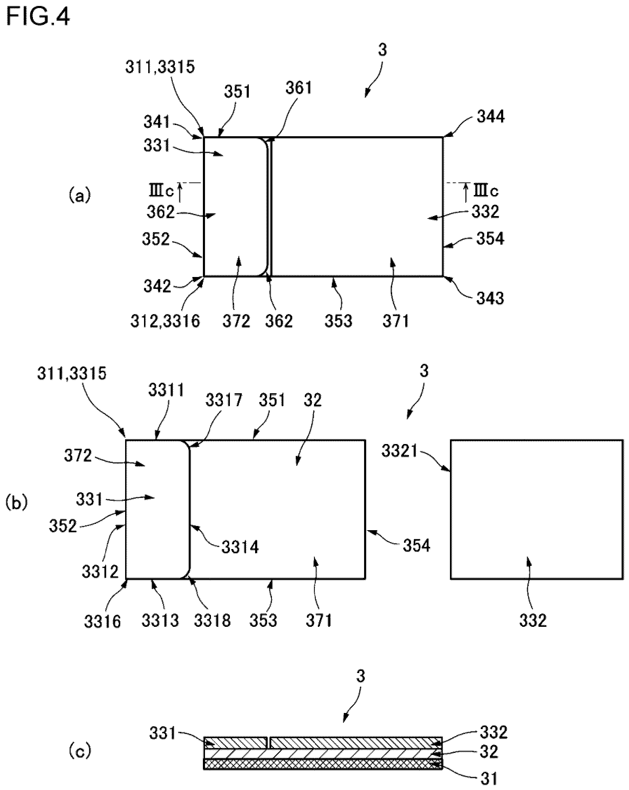

[FIG. 4] FIG. 4 includes diagrams illustrating a configuration of a skin adhesive sheet according to a third embodiment of the invention: FIG. 4(a) is a top view of the entire skin adhesive sheet as viewed from the release liner side, the left side of FIG. 4(b) is a top view of the base layer and covering, the right side of FIG. 4(b) is a top view of the release liner, and FIG. 4(c) is a cross-sectional view taken along line IIIc-IIIc in FIG. 4(a).

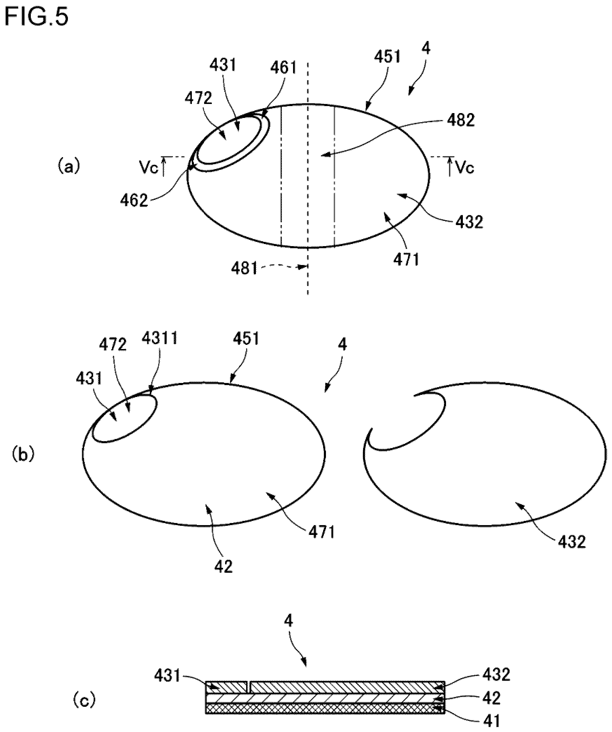

[FIG. 5] FIG. 5 includes diagrams illustrating a configuration of a skin adhesive sheet according to a fourth embodiment of the invention: FIG. 5(a) is a top view of the entire skin adhesive sheet as viewed from the release liner side, the left side of FIG. 5(b) is a top view of the base layer and covering, the right side of FIG. 5(b) is a top view of the release liner, and FIG. 5(c) is a cross-sectional view taken along line Vc-Vc in FIG. 5(a).

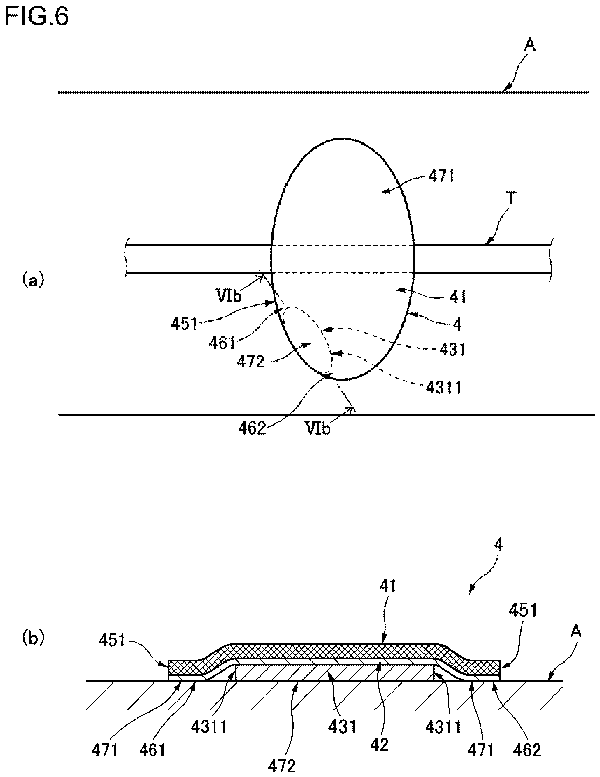

[FIG. 6] FIG. 6 includes diagrams illustrating a state in which the skin adhesive sheet of FIG. 5 is applied to an object: FIG. 6(a) is a top view, and FIG. 6(b) is a cross-sectional view taken along line VIb-VIb in FIG. 6(a).

### Modes for Carrying Out the Invention

Hereinafter, exemplary embodiments of a skin adhesive sheet will be described in detail along with its configuration and operation with reference to the accompanying drawings. It should be noted that the invention is not limited to the embodiments described below. The components or elements described below may include those that are readily conceivable to a person skilled in the art as well as those that are substantially identical. In addition, the components or elements described below may be combined as appropriate.

The skin adhesive sheet of the present disclosure may be used, for example, to secure the needle or tube of a catheter to the skin of a patient or the like.

A first embodiment of the invention will be described below with reference to the drawings.

FIG. 1 includes diagrams illustrating a configuration of a skin adhesive sheet according to the first embodiment: FIG. 1(a) is a top view of the entire skin adhesive sheet as viewed from the release liner side, the left side of FIG. 1(b) is a top view of the base layer and covering, the right side of FIG. 1(b) is a top view of the release liner, and FIG. 1(c) is a cross-sectional view taken along line Ic-Ic in FIG. 1(a). FIG. 2 includes diagrams illustrating a state in which the skin adhesive sheet is applied to an object: FIG. 2(a) is a top view, and FIG. 2(b) is a cross-sectional view taken along line IIb-IIb in FIG. 2(a). In FIG. 1, a covering 131 is in a triangular or near-triangular shape. The near-triangular shape refers to the shape in which any of its corners is/are rounded and the lines connecting the unrounded corner(s) and the intersection(s) of the sides extended from the rounded corner(s) form a triangle. For example, a rounded corner may be a corner with a curvature of 0.1 mm or more.

As illustrated in FIGS. 1 and 2, a skin adhesive sheet 1 of the first embodiment includes a base layer 11, an adhesive 12 provided on a surface on one side (the upper side in FIG. 1(c)) of the base layer 11 and capable of adhering to an object A, and a release liner 132 removably attached to the adhesive 12. As illustrated in FIGS. 1(b) and 2, the skin adhesive sheet 1 has an adhesive region 171 that can adhere to the object A (e.g., the skin of a patient, etc.) when the adhesive 12 is exposed by peeling off the release liner 132 and a non-adhesive region 172 that does not adhere to the object A. Incidentally, FIG. 2 illustrates an example in which a tube (specific item) T is secured to the skin, the object A, using the skin adhesive sheet 1.

As illustrated in the cross-sectional view of FIG. 1(c), the skin adhesive sheet 1 is formed of the base layer 11, the adhesive 12 placed thereon, and the covering 131 and the release liner 132 placed on the adhesive 12.

Generally, a worker removes the release liner 132 from the base layer 11 immediately before using the skin adhesive sheet 1. More specifically, a worker peels off the release liner 132 from the base layer 11 to expose the adhesive 12 on the surface and uses the exposed adhesive 12 to attach the base layer 11 to the skin, i.e., the object A.

The skin adhesive sheet 1 is provided with the covering 131 that is not removed from the base layer 11 during the use of the skin adhesive sheet 1. Meanwhile, the release liner 132 is removed from the base layer 11 before the use of the skin adhesive sheet 1.

That is, the non-adhesive region 172 of this embodiment is formed by the covering 131 that is attached to the base layer 11 through the adhesive 12. The covering 131 is a sheet designed to cover the adhesive 12 and is not removed from the base layer 11 even when the release liner 132 is removed to apply the skin adhesive sheet 1 to the object A. The covering 131 may be made of a sheet of a different material than the release liner 132, or it may be made of the same sheet as the release liner 132. For example, a sheet of release liner may be attached to the base layer 11 through the adhesive 12 and then cut at a predetermined position to form the covering 131 and the release liner 132 from the same sheet.

In the base layer 11 of the skin adhesive sheet 1, the adhesive 12 is not exposed on the surface during the use of the skin adhesive sheet 1 in a portion where the covering 131 is present. Therefore, even if a worker pinches the non-adhesive region 172 covered by the covering 131 with gloved fingers, the skin adhesive sheet 1 will not accidentally adhere to the glove. On the other hand, since the release liner 132 is removed from the base layer 11 before the use of the skin adhesive sheet, the adhesive 12 is exposed in this portion (in the adhesive region 171). Thus, the exposed adhesive 12 can be used to attach the skin adhesive sheet 1 to the skin.

For the above reasons, when using the skin adhesive sheet 1, a worker can remove the skin adhesive sheet 1 from the skin by pinching the non-adhesive region 172 where the covering 131 is present, while avoiding accidental adhesion of the skin adhesive sheet 1 to the glove. In other words, in the use of the skin adhesive sheet 1, the release liner 132 is removed to expose the adhesive 12 to allow the base layer 11 to be adhered to the object with the exposed adhesive 12 while having the non-adhesive region 172, and the non-adhesive region 172 can be pinched with fingers to remove the base layer 11 from the object. In addition, as illustrated in FIG. 2(a), the skin adhesive sheet 1 may be used to secure the specific item T (e.g., a tube) to the object A by adhering the base layer 11 having the non-adhesive region 172 to the object A. In this case, the non-adhesive region 172 can also be pinched with fingers to remove the base layer 11 from the object A so that the specific item T can be removed from the object A. Note that since the covering 131 is attached to the non-adhesive region 172 from the time when the release liner 132 is removed from the adhesive region 171 and the adhesion of the base layer 11 to the object is completed until the non-adhesive region 172 is pinched with fingers to remove the base layer 11 from the object, the adhesive 12 is not exposed in this portion (in the non-adhesive region 172).

As illustrated in the top view of FIG. 1(a), the skin adhesive sheet 1 is rectangular or strip-shaped with four corners in overall form. The four corners include a first corner 141, a second corner 142, a third corner 143, and a fourth corner 144. The base layer 11 has four sides that connect to the corners: a first side 151, a second side 152, a third side 153, and a fourth side 154. The base layer 11 also has a base layer corner 111 that is defined by the first side 151 and the second side 152.

As illustrated in the top view of FIG. 1(b), the covering 131 is in the shape of a right triangle or a nearly right triangle having three sides: a first side 1311, a second side 1312, and a third side 1313. The nearly right triangle refers to a triangle in which a corner (hereinafter referred to as "covering first corner") 1314 between the first side 1311 and the second side 1312 has a curvature of 0.1 mm or more, and two sides (the first side 1311 and the second side 1312) connected to the covering first corner 1314, when extended as straight lines, form a right angle at their intersection.

In the skin adhesive sheet 1, the base layer 11 and the covering 131 are stacked one on top of the other such that the base layer corner 111, which is a right-angled or nearly right-angled corner, and the covering first corner 1314, which is a right-angled or nearly right-angled corner, are aligned at the first corner 141 of the skin adhesive sheet 1. As a result, the first side 151 of the base layer 11 and the first side 1311 of the covering 131 are located to face (overlap) each other. This means that the non-adhesive region 172 of this embodiment is located at a position along the outer edges (the first side 151 and the second side 152) of the base layer 11.

The covering 131 has the covering first corner 1314 that is defined by the first side 1311 and the second side 1312 and aligned with the base layer corner 111, a covering second corner 1315 that is located on the first side 1311 on the opposite side to the covering first corner 1314, and a covering third corner 1316 that is located on the second side 1312 on the opposite side to the covering first corner 1314. The first side 1311 of the covering 131 is located along the first side 151 of the base layer 11, and the second side 1312 of the covering 131 is located along the second side 152 of the base layer 11. In this embodiment, the covering second corner 1315 and the covering third corner 1316 are rounded.

In the skin adhesive sheet 1, a first side protection region (side protection zone) 161, which is part of the adhesive region 171, is provided between the first side 151 of the base layer 11 and the covering second corner 1315 of the covering 131 (see FIG. 2(b)). For example, the first side protection region 161 may be formed by arranging the covering second corner 1315 of the covering 131 at a certain angle relative to the first side 151 of the base layer 11, or by arranging the rounded covering second corner 1315 relative to the straight first side 151. Accordingly, the first side protection region 161 is formed only of the base layer 11 and the adhesive 12 and does not include the covering 131. Due to the first side protection region 161, the covering second corner 1315 does not stick out of the base layer 11 (the first side 151 of the base layer 11) when the skin adhesive sheet 1 is applied to the skin, the object A (see FIG. 2(b)). The first side protection region 161 is located between the covering second corner 1315 of the covering 131 and the skin (skin adjacent to the edge of the skin adhesive sheet 1) when the skin adhesive sheet 1 is applied to the skin, the object A (see FIG. 2(b)). Thus, the first side protection region 161 can prevent the covering second corner 1315 of the covering 131 from touching or rubbing against the skin. As a result, when the skin adhesive sheet 1 is applied to the skin, the object A, the first side protection region 161 alleviates irritation to the object A that may be caused by the covering second corner 1315.

In addition, in this embodiment, a second side protection region (second side protection zone) 162, which is part of the adhesive region 171, is provided between the second side 152 of the base layer 11 and the covering third corner 1316 of the covering 131 (see FIG. 2(b)). For example, the second side protection region 162 may be formed by arranging the covering third corner 1316 of the covering 131 at a certain angle relative to the second side 152 of the base layer 11, or by arranging the rounded covering third corner 1316 relative to the straight second side 152. Accordingly, the second side protection region 162 is formed only of the base layer 11 and the adhesive 12 and does not include the covering 131. Due to the second side protection region 162, the covering third corner 1316 does not stick out of the base layer 11 (the second side 152 of the base layer 11) when the skin adhesive sheet 1 is applied to the skin, the object A (see FIG. 2(b)). The second side protection region 162 is located between the covering third corner 1316 of the covering 131 and the skin (skin adjacent to the edge of the skin adhesive sheet 1) when the skin adhesive sheet 1 is applied to the skin, the object A, (see FIG. 2(b)). Thus, the second side protection region 162 can prevent the covering third corner 1316 of the covering 131 from touching or rubbing against the skin. As a result, when the skin adhesive sheet 1 is applied to the skin, the object A, the second side protection region 162 alleviates irritation to the object A that may be caused by the covering third corner 1316.

The covering second corner 1315, which is a corner where the first side 1311 of the covering 131 is connected and which faces the first side protection region 161, preferably has a radius of curvature of 0.1 mm or more and 5.0 mm or less, more preferably 0.5 mm or more and 5.0 mm or less. Similarly, the covering third corner 1316, which is a corner where the second side 1312 of the covering 131 is connected and which faces the second side protection region 162, preferably has a radius of curvature of 0.1 mm or more and 5.0 mm or less, more preferably 0.5 mm or more and 5.0 mm or less. However, as the radius of curvature of the covering second corner 1315 and the covering third corner 1316 increases, the covering 131 correspondingly increases in size, and the area of the non-adhesive region 172 also increases. This results in a reduction in the area of the adhesive region 171. In order to ensure an appropriate area for the adhesive region 171, it is desirable that the radius of curvature of the covering second corner 1315 and the covering third corner 1316 be 5.0 mm or less.

In this embodiment, the covering second corner 1315 and the covering third corner 1316 of the covering 131 are rounded; however, they need not necessarily be rounded and may be formed by straight lines. For example, the covering second corner 1315 of the covering 131 may be provided by cutting out in a straight manner a corner formed by the first side 1311 and the third side 1313 of the covering 131 extended as straight lines. Similarly, the covering third corner 1316 of the covering 131 may be provided by cutting out in a straight manner a corner formed by the second side 1312 and the third side 1313 of the covering 131 extended as straight lines. In other words, the term "corner" as used herein does not refer to a corner in a general sense but rather refers to a portion located at a position corresponding to a corner of the shape of the covering 131.

As illustrated in FIG. 1, the skin adhesive sheet 1 desirably includes, in addition to the covering 131, the release liner 132 that is removed from the skin adhesive sheet 1 immediately before use. This is because the release liner 132 can be used to protect the adhesive 12 before the use of the skin adhesive sheet 1.

As illustrated in FIGS. 1(a) and 1(b), the release liner 132 has a first side 1321, a second side 1322, a third side 1323, and a fourth side 1324 along the first side 151, the second side 152, the third side 153, and the fourth side 154 of the base layer 11. The release liner 132 also has a fifth side 1325, a sixth side 1326, and a seventh side 1327, which are adjacent to the covering second corner 1315, the covering third corner 1316, and the third side 1313 of the covering 131, respectively. The first side 1321 and the fifth side 1325 of the release liner 132 form a corner 1328, which may be acute-angled. The second side 1322 and the sixth side 1326 of the release liner 132 form a corner 1329, preferably at an acute angle. Since the release liner 132 is removed before the skin adhesive sheet 1 is applied to the skin, the sharp corners 1328 and 1329 of the release liner 132 are eliminated. As a result, there remains only the covering 131 with blunt corners, i.e., the covering second corner 1315 and the covering third corner 1316, which prevents irritation to the skin caused by sharp corners touching or rubbing against the skin.

As illustrated in FIG. 2, the tube (specific item) T is secured to the skin, the object A, by using a region around the center of the skin adhesive sheet 1 in the longitudinal direction. Specifically, as illustrated in FIG. 1(a), a central region 182 (the region between the dash-dot-dot lines in FIG. 1(a)), which includes a center line 181 connecting a first midpoint 1831 at the center of a first long side 183 of the skin adhesive sheet 1 and a second midpoint 1841 at the center of a second long side 184, is used as the adhesive region 171. Therefore, it is desirable that there be no non-adhesive region 172 in the central region 182. The central region 182, which does not include the non-adhesive region 172, desirably has an area of at least one-tenth of the total area of the skin adhesive sheet 1 to secure the tube (specific item) T to the skin adhesive sheet 1. More preferably, the central region 182 has an area of at least eight-tenths of the total area to secure the skin adhesive sheet 1, to which the tube (specific item) T is secured, to the skin, the object A.

A second embodiment of the invention will be described below with reference to the drawings. A skin adhesive sheet 2 of this embodiment differs from that of the first embodiment in that it includes a release liner (132) and a covering (231) having facing sides that are undulated in a wave shape. The following description may omit details of configurations that are similar to those of the first embodiment.

FIG. 3 includes diagrams illustrating a configuration of a skin adhesive sheet according to the second embodiment: FIG. 3(a) is a top view of the entire skin adhesive sheet as viewed from the release liner side, the left side of FIG. 3(b) is a top view of the base layer and covering, the right side of FIG. 3(b) is a top view of the release liner, and FIG. 3(c) is a cross-sectional view taken along line IIc-IIc in FIG. 3(a). In FIG. 2, the covering and release liner have facing sides that are undulated in a wave shape.

As illustrated in FIG. 3, the skin adhesive sheet 2 of the second embodiment includes a base layer 21, an adhesive 22, a covering 231, and a release liner 232. As illustrated in FIG. 3(b), the skin adhesive sheet 2 has an adhesive region 271 that can adhere to an object (e.g., the skin of a patient, etc.) when the adhesive 22 is exposed by peeling off the release liner 232 and a non-adhesive region 272 that does not adhere to the object.

As illustrated in the cross-sectional view of FIG. 3(c), the skin adhesive sheet 2 of this embodiment is formed of the base layer 21, the adhesive 22 placed thereon, and the covering 231 and the release liner 232 placed on the adhesive 22.

The non-adhesive region 272 of this embodiment is formed by the covering 231 that is attached to the base layer 21 through the adhesive 22. The covering 231 is a sheet designed to cover the adhesive 22 and is not removed from the base layer 21 even when the release liner 232 is removed to apply the skin adhesive sheet 2 to an object.

As illustrated in the top view of FIG. 3(a), the skin adhesive sheet 2 is rectangular or strip-shaped with four corners in overall form. The four corners include a first corner 241, a second corner 242, a third corner 243, and a fourth corner 244. The base layer 21 has four sides that connect to the corners: a first side 251, a second side 252, a third side 253, and a fourth side 254. The base layer 21 also has a base layer corner 211 that is defined by the first side 251 and the second side 252.

As illustrated in the top view of FIG. 3(b), the covering 231 is in the shape of a right triangle or a nearly right triangle having three sides: a first side 2311, a second side 2312, and a third side 2313. The nearly right triangle refers to a triangle in which a corner (hereinafter referred to as "covering first corner") 2314 between the first side 2311 and the second side 2312 has a curvature of 0.1 mm or more, and two sides (the first side 2311 and the second side 2312) connected to the covering first corner 2314, when extended as straight lines, form a right angle at their intersection.

In the skin adhesive sheet 2, the base layer 21 and the covering 231 are stacked one on top of the other such that the base layer corner 211 and the covering first corner 2314 are aligned at the first corner 241 of the skin adhesive sheet 2. As a result, the first side 251 of the base layer 21 and the first side 2311 of the covering 231 are located to face (overlap) each other. This means that the non-adhesive region 272 of this embodiment is located at a position along the outer edges (the first side 251 and the second side 252) of the base layer 21.

The covering 231 has the covering first corner 2314 that is defined by the first side 2311 and the second side 2312 and aligned with the base layer corner 211, a covering second corner 2315 that is located on the first side 2311 on the opposite side to the covering first corner 2314, and a covering third corner 2316 that is located on the second side 2312 on the opposite side to the covering first corner 2314. The first side 2311 of the covering 231 is located along the first side 251 of the base layer 21, and the second side 2312 of the covering 231 is located along the second side 252 of the base layer 21. In this embodiment, the covering second corner 2315 and the covering third corner 2316 are rounded.

In the skin adhesive sheet 2, a first side protection region (side protection zone) 261, which is part of the adhesive region 271, is provided between the first side 251 of the base layer 21 and the covering second corner 2315 of the covering 231. Due to the first side protection region 261, the covering second corner 2315 does not stick out of the base layer 21 (the first side 251 of the base layer 21) when the skin adhesive sheet 2 is applied to the skin, an object. Thus, the first side protection region 261 can prevent the covering second corner 2315 of the covering 231 from touching or rubbing against the skin. As a result, when the skin adhesive sheet 2 is applied to the skin, an object, the first side protection region 261 alleviates irritation to the object that may be caused by the covering second corner 2315.

Similarly, a second side protection region (second side protection zone) 262 is provided between the second side 252 of the base layer 21 and the covering third corner 2316 of the covering 231. Due to the second side protection region 262, the covering third corner 2316 does not stick out of the base layer 21 (the second side 252 of the base layer 21) when the skin adhesive sheet 2 is applied to the skin, an object. Thus, the second side protection region 262 can prevent the covering third corner 2316 of the covering 231 from touching or rubbing against the skin. As a result, when the skin adhesive sheet 2 is applied to the skin, an object, the second side protection region 262 alleviates irritation to the object that may be caused by the covering third corner 2316.

As illustrated in FIG. 3, the skin adhesive sheet 2 desirably includes, in addition to the covering 231, the release liner 232 that is removed from the skin adhesive sheet 2 immediately before use. This is because the release liner 232 can be used to protect the adhesive 22 before the use of the skin adhesive sheet 2.

As illustrated in FIGS. 3(a) and 3(b), the release liner 232 has a first side 2321, a second side 2322, a third side 2323, and a fourth side 2324 along the first side 251, the second side 252, the third side 253, and the fourth side 254 of the base layer 21. The release liner 232 also has a fifth side 2325, a sixth side 2326, and a seventh side 2327, which are adjacent to the covering second corner 2315, the covering third corner 2316, and the third side 2313 of the covering 231, respectively. The first side 2321 and the fifth side 2325 of the release liner 232 form a corner 2328, which may be acute-angled. The second side 2322 and the sixth side 2326 of the release liner 232 form a corner 2329, which may also be acute-angled. This is because the release liner 232 is removed before the skin adhesive sheet 1 is applied to the skin, thus eliminating the sharp corners 2328 and 2329 of the release liner 232. As a result, there remains only the covering 231 with blunt corners, i.e., the covering second corner 2315 and the covering third corner 2316, which prevents irritation to the skin caused by sharp corners touching or rubbing against the skin.

In the skin adhesive sheet 2 of this embodiment, it is desirable that the third side 2313 of the covering 231 be provided with a recessed portion 2317, as illustrated in FIG. 3. This allows a raised portion 2330 to be provided on the seventh side 2327 of the release liner 232 adjacent to the third side 2313 of the covering 231. When the skin adhesive sheet 2 is bent at the part between the covering 231 and the release liner 232 with the raised portion 2330, the raised portion 2330 of the release liner 232 is peeled off from the skin adhesive sheet 2. Thus, the raised portion 2330 can be pinched with fingers to remove the release liner 232 from the skin adhesive sheet 2. Conversely, the third side 2313 of the covering 231 may be provided with a raised portion and the seventh side 2327 of the release liner 232 may be provided with a recessed portion so that the release liner 232 can be pinched.

A third embodiment of the invention will be described below with reference to the drawings. A skin adhesive sheet 3 of this embodiment differs from that of the first embodiment in the shapes of its release liner (332) and covering (331). The following description may omit details of configurations that are similar to those of the first embodiment.

FIG. 4 includes diagrams illustrating a configuration of a skin adhesive sheet according to the third embodiment: FIG. 4(a) is a top view of the entire skin adhesive sheet as viewed from the release liner side, the left side of FIG. 4(b) is a top view of the base layer and covering, the right side of FIG. 4(b) is a top view of the release liner, and FIG. 4(c) is a cross-sectional view taken along line IIIc-IIIc in FIG. 4(a). The covering illustrated in FIG. 4 is in a quadrilateral, rectangular or near-rectangular shape. The near-rectangular shape refers to the shape in which any of its corners is/are rounded and the lines connecting the unrounded corner(s) and the intersection(s) of the sides extended from the rounded corner(s) form a quadrilateral or a rectangle. For example, a rounded corner may be a corner with a curvature of 0.1 mm or more.

As illustrated in FIG. 4, the skin adhesive sheet 3 of the third embodiment includes a base layer 31, an adhesive 32, a covering 331, and a release liner 332. As illustrated in FIG. 4(b), the skin adhesive sheet 3 has an adhesive region 371 that can adhere to an object (e.g., the skin of a patient, etc.) when the adhesive 32 is exposed by peeling off the release liner 332 and a non-adhesive region 372 that does not adhere to the object.

As illustrated in the cross-sectional view of FIG. 4(c), the skin adhesive sheet 3 of this embodiment is formed of the base layer 31, the adhesive 32 placed thereon, and the covering 331 and the release liner 332 placed on the adhesive 32.

The non-adhesive region 372 of this embodiment is formed by the covering 331 that is attached to the base layer 31 through the adhesive 32. The covering 331 is a sheet designed to cover the adhesive 32 and is not removed from the base layer 31 even when the release liner 332 is removed to apply the skin adhesive sheet 3 to an object.

As illustrated in the top view of FIG. 4(a), the skin adhesive sheet 3 is rectangular or strip-shaped with four corners in overall form. The four corners include a first corner 341, a second corner 342, a third corner 343, and a fourth corner 344. The base layer 31 has four sides that connect to the corners: a first side 351, a second side 352, a third side 353, and a fourth side 354. The first side 351 and the third side 353 are opposite sides, and the second side 352 and the fourth side 354 are opposite sides. The base layer 31 also has a base layer corner 311 that is defined by the first side 351 and the second side 352.

As illustrated in the top view of FIG. 4(b), the covering 331 of this embodiment is quadrilateral or rectangular in shape and has four sides: a first side 3311, a second side 3312, a third side 3313, and a fourth side 3314.

In the skin adhesive sheet 3, the base layer 31 and the covering 331 are stacked one on top of the other such that the base layer corner 311 and a covering first corner 3315 (a corner defined by the first side 3311 and the second side 3312) are aligned at the first corner 341 of the skin adhesive sheet 3. As a result, the first side 351 of the base layer 31 and the first side 3311 of the covering 331 are located to face (overlap) each other. In addition, a corner 312 of the base layer 31 and a corner 3316 of the covering 331 (a corner defined by the second side 3312 and the third side 3313) are aligned at the second corner 342 of the skin adhesive sheet 3. This means that the non-adhesive region 372 of this embodiment is located at a position along the outer edges (the first side 351, the second side 352, and the third side 353) of the base layer 31. The fourth side 3314 of the covering 331 is adjacent to a first side 3321 of the release liner 332.

In addition to the covering first corner 3315 and the corner 3316, the covering 331 has a covering second corner 3317 that is located on the first side 3311 on the opposite side to the covering first corner 3315, and a covering third corner 1318 that is located on the third side 3313 on the opposite side to the corner 3316. In other words, the covering third corner 1318 is located at a position continuous with the third side 3313 and diagonal to the covering first corner 3315. The first side 3311 of the covering 331 is located along the first side 351 of the base layer 31, the second side 3312 of the covering 331 is located along the second side 352 of the base layer 31, and the third side 3313 of the covering 331 is located along the third side 353 of the base layer 31. In this embodiment, the covering second corner 3317 and the covering third corner 3318 are rounded.

In the skin adhesive sheet 3, a first side protection region (side protection zone) 361, which is part of the adhesive region 371, is provided between the first side 351 of the base layer 31 and the covering second corner 3317 of the covering 331. Due to the first side protection region 361, the covering second corner 3317 does not stick out of the base layer 31 (the first side 351 of the base layer 31) when the skin adhesive sheet 3 is applied to the skin, an object. Thus, the first side protection region 361 can prevent the covering second corner 3317 of the covering 331 from touching or rubbing against the skin. As a result, when the skin adhesive sheet 3 is applied to the skin, an object, the first side protection region 361 alleviates irritation to the object that may be caused by the covering second corner 3317.

Similarly, a second side protection region (second side protection zone) 362 is provided between the third side 353 of the base layer 31 and the covering third corner 3318 of the covering 331. Due to the second side protection region 362, the covering third corner 3318 does not stick out of the base layer 31 (the third side 353 of the base layer 31) when the skin adhesive sheet 3 is applied to the skin, an object. Thus, the second side protection region 362 can prevent the covering third corner 3318 of the covering 331 from touching or rubbing against the skin. As a result, when the skin adhesive sheet 3 is applied to the skin, an object, the second side protection region 362 alleviates irritation to the object that may be caused by the covering third corner 3318.

The covering second corner 3317 of the covering 331, which faces the first side protection region 361, preferably has a radius of curvature of 0.1 mm or more and 5.0 mm or less, more preferably 0.5 mm or more and 5.0 mm or less. Similarly, the covering third corner 3318 of the covering 331, which faces the second side protection region 362, preferably has a radius of curvature of 0.1 mm or more and 5.0 mm or less, more preferably 0.5 mm or more and 5.0 mm or less. However, as the radius of curvature of the covering second corner 3317 and the covering third corner 3318 increases, the covering 331 correspondingly increases in size, and the area of the non-adhesive region 372 also increases. This results in a reduction in the area of the adhesive region 371. In order to ensure an appropriate area for the adhesive region 371, it is desirable that the radius of curvature of the covering second corner 3317 and the covering third corner 3318 be 5.0 mm or less. In addition, the area of the non-adhesive region 372 is preferably one-third or less, and more preferably one-tenth or less, of that of the adhesive region 371 to achieve sufficient adhesive strength.

A fourth embodiment of the invention will be described below with reference to the drawings. A skin adhesive sheet 4 of this embodiment differs in shape from that of the first embodiment. The following description may omit details of configurations that are similar to those of the first embodiment.

FIG. 5 includes diagrams illustrating a configuration of a skin adhesive sheet according to the fourth embodiment: FIG. 5(a) is a top view of the entire skin adhesive sheet as viewed from the release liner side, the left side of FIG. 5(b) is a top view of the base layer and covering, the right side of FIG. 5(b) is a top view of the release liner, and FIG. 5(c) is a cross-sectional view taken along line Vc-Vc in FIG. 5(a). FIG. 6 includes diagrams illustrating a state in which the skin adhesive sheet of FIG. 5 is applied to an object: FIG. 6(a) is a top view, and FIG. 6(b) is a cross-sectional view taken along line VIb-VIb in FIG. 6(a).

As illustrated in FIG. 5, the skin adhesive sheet 4 of the fourth embodiment includes an elliptical base layer 41, an adhesive 42, an elliptical covering 431, and a release liner 432.

As illustrated in FIG. 5(b), the skin adhesive sheet 4 has an adhesive region 471 that can adhere to an object (e.g., the skin of a patient, etc.) when the adhesive 42 is exposed by peeling off the release liner 432 and a non-adhesive region 472 that does not adhere to the object.

As illustrated in the cross-sectional view of FIG. 5(c), the skin adhesive sheet 4 of this embodiment is formed of the base layer 41, the adhesive 42 placed thereon, and the covering 431 and the release liner 432 placed on the adhesive 42.

As illustrated in FIGS. 5(a) and 6, in the skin adhesive sheet 4, a first side protection region (side protection zone) 461, which is part of the adhesive region 471, is provided between a periphery 451 of the base layer 41 and a region located on one side of a periphery 4311 of the covering 431 in the longitudinal direction. Due to the first side protection region 461, the region located on one side of the periphery 4311 of the covering 431 in the longitudinal direction does not stick out of the base layer 41 (the periphery 451 of the base layer 41) when the skin adhesive sheet 4 is applied to the skin, an object A. Thus, the first side protection region 461 can prevent the region located on one side of the periphery 4311 of the covering 431 in the longitudinal direction from touching or rubbing against the skin. As a result, when the skin adhesive sheet 4 is applied to the skin, the object A, the first side protection region 461 alleviates irritation to the object A that may be caused by the region located on one side of the periphery 4311 of the covering 431 in the longitudinal direction.

Similarly, a second side protection region 462 is provided between the periphery 451 of the base layer 41 and a region located on the other side of the periphery 4311 of the covering 431 in the longitudinal direction. Due to the second side protection region 462, the region located on the other side of the periphery 4311 of the covering 431 in the longitudinal direction does not stick out of the base layer 41 (the periphery 451 of the base layer 41) when the skin adhesive sheet 4 is applied to the skin, the object A. Thus, the second side protection region 462 can prevent the region located on the other side of the periphery 4311 of the covering 431 in the longitudinal direction from touching or rubbing against the skin. As a result, when the skin adhesive sheet 4 is applied to the skin, the object A, the second side protection region 462 alleviates irritation to the object A that may be caused by the region located on the other side of the periphery 4311 of the covering 431 in the longitudinal direction.

As illustrated in FIG. 6(a), the skin adhesive sheet 4 may be used to secure a specific item T (e.g., a tube) to the object A by adhering the base layer 41 having the non-adhesive region 472 to the object A. In this case, the non-adhesive region 472 can also be pinched with fingers to remove the base layer 41 from the object A so that the specific item T can be removed from the object A.

As illustrated in FIG. 5( a ), when the skin adhesive sheet 4 has an elliptical or nearly elliptical shape, a region around a minor axis 481 of the skin adhesive sheet 4 is used to secure the tube (specific item) T to the skin, the object A. In other words, a region around the minor axis 481 of the skin adhesive sheet 4 is used as the adhesive region 471. Therefore, it is desirable that there be no non-adhesive region 472 in a central region 482 (the region between the dash-dot-dot lines in FIG. 5(a)) including the minor axis 481. The central region 482, which does not include the non-adhesive region 472, desirably has an area of at least one-tenth of the total area of the skin adhesive sheet 4 to secure the tube (specific item) T to the skin adhesive sheet 1. More preferably, the central region 482 has an area of at least eight-tenths of the total area to secure the skin adhesive sheet 1, to which the tube (specific item) T is secured, to the skin, the object A.

The materials of the individual members or elements will be described below.

The base layer may be made of material commonly used for surgical tapes and dressings. The material may be a non-woven fabric or a resin film, preferably the one used for medical devices. The thickness of the base layer is preferably 500 µm or less, more preferably 100 µm or less.

The adhesive may be made of material commonly used for surgical tapes and dressings. The material may be acrylic-based or silicone-based, preferably adhesive material for medical devices that is less irritating to the skin. The thickness of the adhesive is preferably 100 µm or less, more preferably 50 µm or less.

The release liner may be made of material commonly used for surgical tapes and dressings. The material may be paper, a non-woven fabric, or a resin film, which is coated with a release agent for medical devices. The thickness of the release liner is preferably 500 µm or less, more preferably 100 µm or less. The covering may be the same as or different in material and thickness from the release liner.

The area of the skin adhesive sheet is preferably, but not limited to, 100 square centimeters or less, more preferably 50 square centimeters or less.

The area of the covering is preferably, but not limited to, 50 square centimeters or less, more preferably 10 square centimeters or less.

The area of the side protection region (side protection zone) is preferably, but not limited to, 5 square centimeters or less, more preferably 1 square centimeter or less.

In the first to third embodiments described above, the non-adhesive region (172, 272, 372) is formed by the covering (131, 231, 331) that is attached to the base layer (11, 21, 31) through the adhesive (12, 22, 32); however, the non-adhesive region (172, 272, 372) is not so limited and may be any region that does not adhere to an object during use. For example, the non-adhesive region (172, 272, 372) may be a region without the adhesive (12, 22, 32).

The following is a description of the two tests that were performed.

In the first test, the skin adhesive sheet 1 of the first embodiment was prepared as follows. First, a base layer with adhesive was cut into a rectangular strip of 5 cm in length and 3 cm in width. Materials used in commercially available skin adhesive sheets were used for the base layer and adhesive. Specifically, the nonwoven fabric base and acrylic adhesive used in Kino White, an adhesive bandage manufactured by Nitoms, Inc., were used.

A release liner and covering were attached to the strip-shaped base layer prepared as above. The release liner and covering were made of the same paper as that used in the Kino White adhesive bandage. First, a strip of release liner measuring 5 cm in length and 3 cm in width was prepared. Next, a right-angled triangular covering with a base of 2 cm and a height of 1 cm was cut out from the strip of release liner. The two corners other than the right-angle corner were provided with a radius of curvature of 0.1 mm or more (0.5 mm). The remaining portion of the strip after cutting out the covering was used as a release liner to be peeled off at the time of use.

The covering was attached to the base layer such that its right-angle corner was aligned with the first corner of the base layer. The release liner was then placed facing the covering.

When the skin adhesive sheet 1 was prepared as described above, two side protection zones were formed around the two corners of the covering other than the right-angle corner. In other words, a side protection zone was formed between one of the first and second sides, which form the first corner of the base layer, and a corresponding one of the two corners of the covering other than the right-angle corner. The side protection zones were formed of the adhesive and the base layer, without the covering.

Test sample 1 was prepared as follows. In the same manner as described above for the skin adhesive sheet 1, the base layer was cut into a rectangular strip, and a release liner and a covering were attached thereto. The covering was in the shape of a right-angled triangle with a base of 2 cm and a height of 1 cm. However, the two corners other than the right-angle corner were made pointed, and their radius of curvature was 0.1 mm or less (0.01 mm).

Test sample 2 was prepared as follows. In the same manner as described above for the skin adhesive sheet 1, the base layer was cut into a rectangular strip, and a right-angled triangular covering was attached thereto. In test sample 2, however, portions corresponding to the side protection zones of the skin adhesive sheet 1 were left uncoated with adhesive and consisted only of the base layer.

10 skin adhesive sheets 1, 10 sheets of test sample 1, and 10 sheets of test sample 2 were prepared and applied to the upper arms of 10 subjects, and the subjects' skin condition was observed 24 hours later. Test sample 1 caused redness or itching on the skin of 5 subjects. Similarly, test sample 2 caused redness or itching on the skin of 6 subjects. In contrast, no subject experienced redness or itching with the skin adhesive sheet 1.

In the second test, the skin adhesive sheet 2 of the second embodiment was prepared as follows. First, a base layer with adhesive was cut into a rectangular strip of 5 cm in length and 3 cm in width. Materials used in commercially available skin adhesive sheets were used for the base layer and adhesive. Specifically, the nonwoven fabric base and acrylic adhesive used in Kino White, an adhesive bandage manufactured by Nitoms, Inc., were used.

A release liner and covering were prepared as follows. The release liner and covering were made of the same materials as those of the Kino White adhesive bandage. First, a strip of release liner measuring 5 cm in length and 3 cm in width was prepared. Next, a covering was cut out from the strip of release liner: the covering was in the shape of a nearly right triangle with a base of 2 cm and a height of 1 cm. The two corners other than the right-angle corner were provided with a radius of curvature of 0.1 mm or more (0.5 mm). In addition, the diagonal side (hypotenuse) was made undulated in a wave shape. Specifically, the diagonal side was provided with three inflection points to create the wave shape. The remaining portion of the strip after cutting out the covering was used as a release liner to be peeled off at the time of use. Since the diagonal side of the covering has a wave shape, the side of the release liner facing the covering was also undulated in a wave shape.

The covering and release liner were attached to the base layer. First, the covering was placed such that its right-angle corner was aligned with the first corner of the base layer, and then the release liner was placed facing the covering.

In the skin adhesive sheet 2, the diagonal side of the covering and the side of the release liner facing the covering are undulated in a wave shape. Therefore, when the skin adhesive sheet is bent between the covering and the release liner, some of the waviness of the release liner is peeled off from the skin adhesive sheet. This facilitates the removal of the release liner from the skin adhesive sheet.

10 skin adhesive sheets 2 were prepared and applied to the upper arms of 10 subjects, and the subjects' skin condition was observed 24 hours later. As a result, no subject experienced redness or itching with the skin adhesive sheet 2.

While preferred embodiments of the present invention have been described and illustrated, the invention is not limited to the embodiments disclosed herein. Various changes, modifications, and alterations may be made within the scope of the invention as defined in the appended claims. In other words, alternative embodiments, examples, and operational techniques made by those skilled in the art based on the foregoing embodiments are within the scope of the present invention.

### Industrial Applicability

The present invention relates to a skin adhesive sheet used to secure an item, such as a medical device, to a patient's body. Therefore, the invention may be suitable for use in the medical device industry.

### List of Reference Signs

1, 2, 3: Skin adhesive sheet
11, 21, 31, 41: Base layer
111, 211, 311: Base layer corner
12, 22, 32, 42: Adhesive
131, 231, 331, 431: Covering
1311, 2311, 3311: First side of covering
1312, 2312, 3312: Second side of covering
1314, 2314, 3315: Covering first corner
1315, 2315, 3317: Covering second corner
1316, 2316, 3318: Covering third corner
132, 232, 332, 432: Release liner
151, 251, 351: First side of base layer
152, 252, 352: Second side of base layer
153, 253, 353: Third side of base layer
161, 261, 361, 461: First side protection region (side protection zone)
162, 262, 362: Second side protection region (second side protection zone)
171, 271, 371, 471: Adhesive region
172, 272, 372, 472: Non-adhesive region
181: Center line
182: Central region including center line
183: First long side
1831: First midpoint
184: Second long side
1841: Second midpoint
2317: Recessed portion of covering
2330: Raised portion of release liner
3313: Third side of covering
4311: Periphery of covering
451: Periphery of base layer
481: Minor axis
482: Central region including minor axis

## Claims

1. A skin adhesive sheet, comprising:
a base layer;
an adhesive that is provided on a surface on one side of the base layer and can adhere to an object; and
a release liner that is removably attached to the adhesive, wherein
the skin adhesive sheet has an adhesive region that can adhere to the object when the adhesive is exposed by removal of the release liner and a non-adhesive region that does not adhere to the object,
the release liner is removed to expose the adhesive so that the base layer can be adhered to the object with the exposed adhesive while having the non-adhesive region,
the adhesive region has a higher adhesive strength than the non-adhesive region after adhesion is complete, and
the non-adhesive region can be pinched with fingers to remove the base layer from the object.

2. The skin adhesive sheet according to claim 1, wherein
the base layer is adhered to the object while having the non-adhesive region to thereby secure a specific item to the object, and
the non-adhesive region can be pinched with fingers to remove the base layer from the object, allowing the specific item to be removed from the object.

3. The skin adhesive sheet according to claim 1 or 2, wherein the non-adhesive region is located at a position along outer edges of the base layer.

4. The skin adhesive sheet according to claim 1 or 2, wherein
the non-adhesive region is formed by a covering that is attached to the base layer through the adhesive, and
the covering is not removed from the base layer when the release liner is removed to apply the skin adhesive sheet to the object.

5. The skin adhesive sheet according to claim 1 or 2, wherein
the skin adhesive sheet is rectangular, nearly rectangular, strip-shaped, or nearly strip-shaped, and
the non-adhesive region is not present in a central region that includes a center line connecting a first midpoint of a first long side of the skin adhesive sheet and a second midpoint of a second long side of the skin adhesive sheet.

6. The skin adhesive sheet according to claim 1 or 2, wherein
the skin adhesive sheet is elliptical or nearly elliptical, and
the non-adhesive region is not present in a central region that includes a minor axis of the skin adhesive sheet.

7. The skin adhesive sheet according to claim 4, wherein
the base layer has a first side, a second side, and a base layer corner defined by the first side and the second side,
the covering has a first side to be along the first side of the base layer, a second side to be along the second side of the base layer, a covering first corner defined by the first side and the second side and to be aligned with the base layer corner, and a covering second corner located opposite the covering first corner on the first side,
the adhesive region includes a side protection zone as part thereof in between the covering second corner and the first side of the base layer, and
the side protection zone reduces irritation to the object caused by the covering second corner when the skin adhesive sheet is applied to the object.

8. The skin adhesive sheet according to claim 7, wherein the covering second corner is rounded.

9. The skin adhesive sheet according to claim 8, wherein the covering second corner has a radius of curvature of 0.1 mm or more and 5.0 mm or less.

10. The skin adhesive sheet according to claim 7, wherein
the covering is triangular in shape,
the covering further has a covering third corner located opposite the covering first corner on the second side,
the covering third corner has a radius of curvature of 0.1 mm or more and 5.0 mm or less,
the adhesive region further includes a second side protection zone as part thereof in between the covering third corner and the second side of the base layer, and
the second side protection zone reduces irritation to the object caused by the covering third corner when the skin adhesive sheet is applied to the object.

11. The skin adhesive sheet according to claim 7, wherein
the base layer further has a third side opposite the first side,
the covering is rectangular in shape,
the covering further has a third side to be along the third side of the base layer, and a covering third corner located at a position continuous with the third side and diagonal to the covering first corner,
the covering third corner has a radius of curvature of 0.1 mm or more and 5.0 mm or less,
the adhesive region further includes a second side protection zone as part thereof in between the covering third corner and the third side of the base layer, and
the second side protection zone reduces irritation to the object caused by the covering third corner when the skin adhesive sheet is applied to the object.

12. The skin adhesive sheet according to claim 7, wherein a side of the covering facing a side of the release liner has a recessed portion or a raised portion.
